Europäisches Patentamt

European Patent Office

Office européen des brevets

(19)

(11) Veröffentlichungsnummer: **0 154 677**
**B1**

(12) # EUROPÄISCHE PATENTSCHRIFT

(45) Veröffentlichungstag der Patentschrift:
22.04.87

(51) Int. Cl.⁴: **C 07 C 15/00**, C 07 C 7/08 //
C10G21/20

(21) Anmeldenummer: 84112017.3

(22) Anmeldetag: 06.10.84

(54) Verfahren zur Abtrennung von Aromaten aus Kohlenwasserstoffgemischen beliebigen Aromatengehaltes.

(30) Priorität: 13.03.84 DE 3409030

(43) Veröffentlichungstag der Anmeldung:
18.09.85 Patentblatt 85/38

(45) Bekanntmachung des Hinweises auf die Patenterteilung:
22.04.87 Patentblatt 87/17

(84) Benannte Vertragsstaaten:
AT BE DE FR GB IT NL

(56) Entgegenhaltungen:
DE - A - 1 444 357
DE - A - 1 568 940

(73) Patentinhaber: Krupp Koppers GmbH, Altendorfer Strasse 120, D-4300 Essen 1 (DE)

(72) Erfinder: Preusser, Gerhard, Dr. Dipl.-Chem., Lönsberg 24, D-4300 Essen 1 (DE)
Erfinder: Schulze, Martin, Krüdenscheider Weg 83, D-5604 Nevlges (DE)

## Beschreibung

Die Erfindung betrifft ein Verfahren zur Abtrennung von Aromaten aus Kohlenwasserstoffgemischen beliebigen Aromatengehaltes, die als nichtaromatische Bestandteile insbesondere Paraffine, Cycloparaffine, Olefine, Diolefine sowie organische Schwefelverbindungen enthalten können, durch Extraktivdestillation, bei der N-substituierte Morpholine, deren Substituenten nicht mehr als sieben C-Atome aufweisen, als selektives Lösungsmittel verwendet werden, wobei die nichtaromatischen Bestandteile des als Einsatzprodukt dienenden Kohlenwasserstoffgemisches als Raffinat über Kopf aus der Extraktivdestillationskolonne abdestilliert werden, während die Aromaten zusammen mit dem verwendeten Lösungsmittel als Extrakt aus dem Sumpf der Extraktivdestillationskolonne abgezogen werden und wobei das Raffinat zwecks Rückgewinnung der in ihm vorhandenen Lösungsmittelreste destilliert wird.

Das vorstehend beschriebene Aromatengewinnungsverfahren ist bereits seit einer ganzen Reihe von Jahren bekannt und hat sich, insbesondere unter Verwendung von N-Formylmorpholin als selektivem Lösungsmittel, in der Zwischenzeit in der Praxis auf verschiedenen grosstechnischen Anlagen gut bewährt. Hierbei wird normalerweise das aus der Extraktivdestillationskolonne abgezogene Sumpfprodukt in eine nachgeschaltete Abtreiberkolonne eingeleitet, in der die in ihm als Extrakt enthaltenen Aromaten destillativ vom Lösungsmittel abgetrennt werden. Das Lösungsmittel wird sodann aus dem Sumpf der Abtreiberkolonne abgezogen und zur Wiederverwendung in die Extraktivdestillationskolonne zurückgeführt. Hierbei erfolgt aus verfahrenstechnischen Gründen die Einleitung bzw. Wiedereinleitung des Lösungsmittels normalerweise am Kopf der Extraktivdestillationskolonne. Dadurch lässt es sich jedoch praktisch nicht vermeiden, dass das anfallende Raffinat noch gewisse Lösungsmittelreste enthält, wobei der Lösungsmittelgehalt im Raffinat bis zu 2 Gew.-% betragen kann. Aus Wirtschaftlichkeitsgründen und im Hinblick auf die Gewinnung eines möglichst reinen Raffinates ist es jedoch unerlässlich, diesen Lösungsmittelanteil im Raffinat möglichst weitgehend zurückzugewinnen.

Dies wäre sicher möglich, wenn man die Extraktivdestillationskolonne mit einem entsprechend hohen Raffinatrückfluss betreiben würde. Im Gegensatz zur normalen Destillation ist jedoch bei der Extraktivdestillation ein derartiger Rückfluss aus folgenden Gründen unangebracht und deshalb zu vermeiden:

1. Ein Raffinatrückfluss führt zu einer Verdünnung des Lösungsmittels und damit zu einer Selektivitätsminderung, wodurch die gewünschte Stofftrennung unnötig erschwert wird.

2. Hochselektive Lösungsmittel — und hierzu gehören die eingangs genannten N-substituierten Morpholine — weisen nur ein begrenztes Lösevermögen für die abzutrennenden nichtaromatischen Kohlenwasserstoffe auf. Ein Raffinatrückfluss kann deshalb dazu führen, dass sich auf den oberen Böden der Extraktivdestillationskolonne zwei Flüssigphasen mit unterschiedlichen Dichten ausbilden, die einen störungsfreien Betrieb der Extraktivdestillationskolonne unmöglich machen.

Daher scheidet dieser an sich naheliegende Weg für die Rückgewinnung des Lösungsmittelanteiles aus dem Raffinat aus, und es muss statt dessen eine separate Rückgewinnung des Lösungsmittels aus dem Raffinat erfolgen. Diese wurde bisher durch einfache Destillation des Raffinates in der Weise durchgeführt, dass das Raffinat als Kopfprodukt mit Lösungsmittelgehalten von < 10 ppm aus der Destillationskolonne abgezogen wurde, während das auf fast 100%ige Reinheit aufkonzentrierte Lösungsmittel aus dem Sumpf dieser Kolonne abgezogen und in die Extraktivdestillationskolonne zurückgeführt wurde.

Diese Arbeitsweise, bei der eine möglichst vollständige Trennung von Raffinat und Lösungsmittel angestrebt wird, erfordert jedoch einen hohen apparativen Aufwand (Destillationskolonne mit hoher Bodenzahl) und einen hohen Energieverbrauch.

Der Erfindung liegt deshalb die Aufgabe zugrunde, das vorstehend beschriebene Verfahren zur destillativen Aufarbeitung des Raffinates zum Zwecke der Abtrennung und Wiedergewinnung des darin enthaltenen Lösungsmittels dahingehend zu verbessern, dass dasselbe mit einem geringeren apparitiven Aufwand und insbesondere auch mit geringerem Energieverbrauch durchgeführt werden kann.

Diese Aufgabe wird gelöst durch ein Verfahren der eingangs genannten Art, das erfindungsgemäss dadurch gekennzeichnet ist, dass das bei der Raffinatdestillation anfallende Sumpfprodukt mit einem Lösungsmittelgehalt von 20 bis 75 Gew.-% aus der Raffinatdestillationskolonne abgezogen und unter Abkühlung bis auf eine Temperatur von 20-70° C in einen Scheidebehälter eingeleitet und dort in eine schwere und eine leichte Phase getrennt wird, worauf die schwere Phase in die Extraktivdestillationskolonne und die leichte Phase in die Raffinatdestillationskolonne wiedereingeleitet wird.

Das heisst, beim erfindungsgemässen Verfahren wird bewusst darauf verzichtet, die Aufkonzentrierung des Lösungsmittels bei der Raffinatdestillation bis auf eine Reinheit von praktisch 100% zu treiben. Eine derartig vollständige Trennung von Raffinat und Lösungsmittel erfordert nämlich bei den zwischen 200 und 280° C liegenden Siedepunkten der eingangs genannten Lösungsmittel einen Beheizungsdampf für die Raffinatdestillationskolonne von mindestens 40-50 bar. Statt dessen sieht das erfindungsgemässe Verfahren vor, die Raffinatdestillation nur so weit zu treiben, bis sich im Sumpfprodukt der Raffinatdestillationskolonne ein Lösungsmittelgehalt zwischen 20 und 75 Gew.-% eingestellt hat. Eine direkte Rückführung dieses Sumpfproduktes in die Extraktivdestillationskolonne wäre allerdings mit beträchtlichen Nachteilen verbunden, da sich in ihm neben dem Lösungsmittel und den im Raffinat als Verunreinigung enthaltenen Aromaten gerade jene nichtaromatischen Kohlenwasserstoffe des Raffinates anreichern, die auf Grund ihrer Siedepunktslage und ihrer chemischen Struktur am schwersten in der Extraktivdestillationskolonne von den Aromaten zu trennen sind und die deshalb ein besonders hohes Verhältnis von Lösungsmittel zu Einsatzprodukt und einen erhöhten Heizenergiebedarf für die Extraktivdestillation erfordern. Hierbei handelt es sich je nach Zusammensetzung des Einsatzproduktes in erster Linie um das Methylcyclohexan, bestimmte Isomere des Dimethylcyclohexans sowie Ethylcyclohexan.

Völlig überraschend wurde nun jedoch gefunden, dass sich das Sumpfprodukt mit dem weiter oben genannten Lösungsmittelgehalt nach Abkühlung auf eine Temperatur von 20-70° C in einem Scheidebehälter in zwei Phasen trennt. Hierbei reichern sich in der leichten Phase (Oberphase) die vorstehend genannten kritischen Kohlenwasserstoffe an, während die schwere Phase (Unterphase) im wesentlichen aus dem Lösungsmittel und den Aromaten besteht, die als Verunreinigungen in das Raffinat gelangt sind. Wegen der bestehenden Dichteunterschiede stellt sich dabei im Scheidebehälter eine scharfe Trennung der beiden Phasen ein. Auf Grund ihrer günstigen Zusammensetzung kann deshalb die schwere Phase in die Extraktivdestillationskolonne wiedereingeleitet werden, ohne dass die weiter oben aufgezeigten Nachteile für den Betrieb derselben eintreten. Die leichte Phase wird dagegen in die Raffinatdestillationskolonne wiedereingeleitet. Die Wiedereinleitung erfolgt hierbei vorzugsweise in den Sumpf der Raffinatdestillationskolonne. Die beschriebene Arbeitsweise kann dabei als kontinuierliches Verfahren ausgeführt werden, wobei die Menge der umlaufenden leichten Phase ein Vielfaches der Menge der abgezogenen schweren Phase beträgt.

Die Behandlung des Raffinates von Extraktivdestillationsprozessen in einem Scheidebehälter ist zwar im Prinzip bekannt, z.B. aus der DE-OS 1 444 357. Bisher diente eine solche Behandlung jedoch ausschliesslich dem Zweck, das Wasser, das infolge der Verwendung von wasserhaltigen Lösungsmitteln in das Raffinat gelangt ist, von den Kohlenwasserstoffen des Raffinates abzutrennen. Diese bekannte Arbeitsweise vermochte deshalb für das Zustandekommen des erfindungsgemässen Verfahrens keinerlei Anregungen zu vermitteln.

Der Lösungsmittelgehalt des Sumpfproduktes der Raffinatdestillation kann über die Sumpftemperatur bzw. die Temperatur der Kolonnenbeheizung am Sumpf der Raffinatdestillationskolonne gesteuert werden, da zwischen dem Lösungsmittelgehalt und der Sumpftemperatur eine eindeutige Beziehung derart besteht, dass mit steigendem Lösungsmittelgehalt die Sumpftemperatur ansteigt, wobei die sich einstellende Sumpftemperatur natürlich von der Siedetemperatur des verwendeten Lösungsmittels und der Zusammensetzung des in der Raffinatdestillationskolonne aufzuarbeitenden Kohlenwasserstoffgemisches abhängig ist. So stellt sich beispielsweise bei der Gewinnung von Benzol aus einer aus Pyrolysebenzin gewonnenen Rohbenzolfraktion durch Extraktivdestillation mit N-Formylmorpholin bei einem Lösungsmittelgehalt im Sumpfprodukt der Raffinatdestillationskolonne von 50 Gew.-% eine Sumpftemperatur von ca. 100° C ein. Liegt dagegen der Gehalt des gleichen Lösungsmittels im Sumpfprodukt bei 75 Gew.-%, so beträgt die Sumpftemperatur ca. 125° C. Selbstverständlich können anstelle der Temperaturmessung auch analytische Methoden, wie z.B. die Gaschromatographie, zur Ermittlung und Kontrolle des Lösungsmittelgehaltes des Sumpfproduktes herangezogen werden.

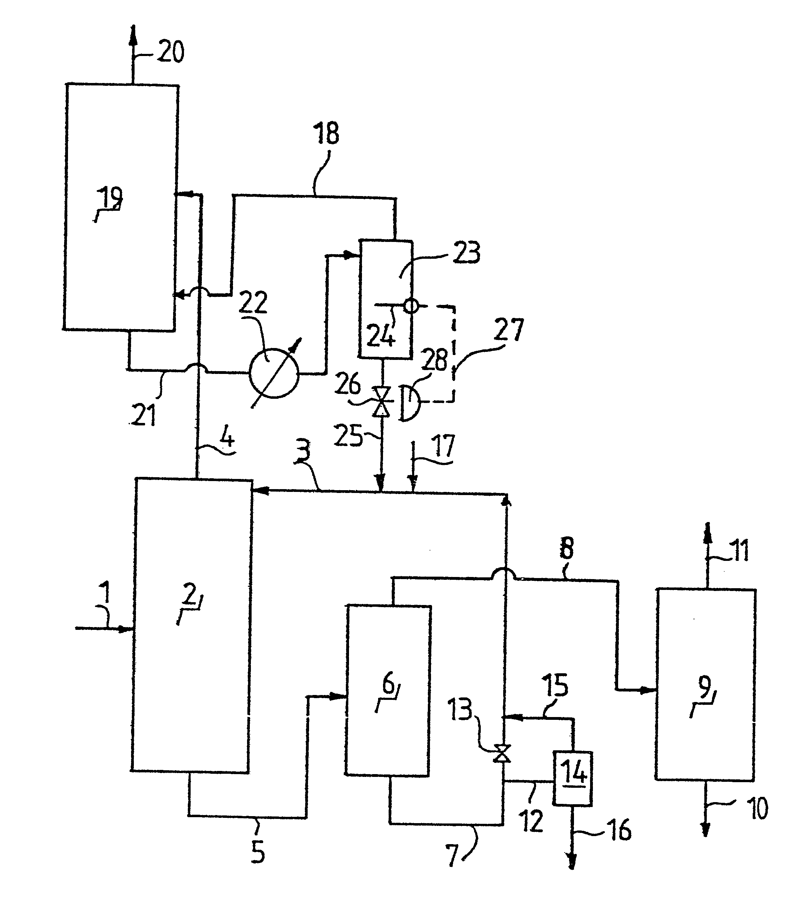

Weitere Einzelheiten des erfindungsgemässen Verfahrens ergeben sich aus den vorliegenden Unteransprüchen und sollen an Hand des in der Abbildung dargestellten Fliessschemas näher erläutert werden. Das Fliessschema enthält dabei nur die für die Verfahrenserläuterung unbedingt erforderlichen Anlagenteile, während Nebeneinrichtungen wie beispielsweise Pumpen, Umlaufkocher, Wärmetauscher etc. nicht dargestellt sind.

Das als Einsatzprodukt dienende Kohlenwasserstoffgemisch, das gegebenenfalls bereits einer Vordestillation unterworfen worden sein kann, wird durch die Leitung 1 in den mittleren Teil der mit Böden versehenen Extraktivdestillationskolonne 2 eingeleitet. Das Einsatzprodukt ist dabei entweder bis dicht unterhalb des Siedepunktes erhitzt, so dass es beim Eintritt in die Extraktivdestillationskolonne sofort verdampft. Es kann aber auch bereits im verdampften Zustand in die Extraktivdestillationskolonne eingeführt werden. Durch die Leitung 3 wird das verwendete selektive Lösungsmittel am Kopf in die Extraktivdestillationskolonne 2 eingeleitet und fliesst über die Böden dieser Kolonne herab nach unten, wobei es die dampfförmigen Aromaten aufnimmt. Die nichtaromatischen Kohlenwasserstoffe, die die Raffinatphase bilden, entweichen durch Leitung 4 am Kopf der Kolonne und gelangen über diese Leitung in den mittleren Teil der mit Füllkörpern oder Böden versehenen Raffinatdestillationskolonne 19.

Das flüssige Sumpfprodukt der Extraktivdestillationskolonne 2 besteht aus dem Lösungsmittel und den darin gelösten Aromaten und wird durch die Leitung 5 aus der Extraktivdestillationskolonne 2 abgezogen und gelangt in die Abtreiberkolonne 6, in der die Aromaten destillativ vom selektiven Lösungsmittel abgetrennt werden. Das Lösungsmittel wird durch die Leitung 7 aus dem Kolonnensumpf entfernt und fliesst über die Leitung 3 wieder in die Extraktivdestillationskolonne 2 zurück, während die Aromaten über Kopf aus der Abtreiberkolonne 6 entweichen und durch die Leitung 8 in die Kolonne 9 gelangen, in der ihre weitere Auftrennung erfolgt. So können beispielsweise durch die Leitung 10 die höhersiedenden Bestandteile und durch die Leitung 11 die niedrigersiedenden Bestandteile abgezogen werden. Da sich im Laufe der Zeit im verwendeten Lösungsmittel Verunreinigungen anreichern können, ist im Bereich der Leitung 7 die Abzweigleitung 12 vorgesehen, durch die bei entsprechender Stellung des Ventils 13 eine Teilmenge des Lösungsmittels zur Regeneriereinrichtung 14 gelangen kann. Das regenerierte Lösungsmittel wird durch die Leitung 15 wieder in den Kreislauf (Leitung 7) zurückgeführt, während die ausgeschiedenen Verunreinigungen durch die Leitung 16 aus der Regeneriereinrichtung entfernt werden. Die Leitung 17 dient schliesslich der Zufuhr von frischem Lösungsmittel.

Zur Durchführung des erfindungsgemässen Verfahrens wird das in der Raffinatdestillationskolonne 19 anfallende Sumpfprodukt mit einem Lösungsmittelgehalt von 20 bis 75 Gew.-% über die Leitung 21 abgezogen, während Kohlenwasserstoffe des Raffinates mit einem Lösungsmittelgehalt unter 10 ppm über die Leitung 20 aus der Raffinatdestillationskolonne 19 entfernt werden. Das Sumpfprodukt in der Leitung 21 gelangt über den Kühler 22, in dem es die erforderliche Abkühlung bis auf eine Temperatur von 20-70° C erfährt, in den Scheidebehälter 23. Dabei tritt das Sumpfprodukt tangential in den Oberteil des Scheidebehälters 23 ein, in dem im mittleren Bereich der Trennschichtregler 24 installiert ist. Da die über die Leitung 21 abfliessende Sumpfproduktmenge verhältnismässig klein ist, wird für deren erforderliche Abkühlung nicht in jedem Falle der Kühler 22 notwendig sein. Gegebenenfalls ist es vielmehr auch möglich, auf diesen Kühler zu verzichten und die Abkühlung des Sumpfproduktes in der Leitung 21 und im Scheidebehälter 23 dadurch vorzunehmen, dass dieselben nicht isoliert sind bzw. mit einem Kühlmantel ausgestattet sind. Eine zu starke Abkühlung des Sumpfproduktes auf eine Temperatur unter 20° C ist nicht angebracht, weil dadurch der Heizenergiebedarf in der Raffinatdestillationskolonne 19 und der Extraktivdestillationskolonne 2 unnötig erhöht werden würde. Bei einer Temperatur zwischen 20 und 70° C erfolgt im Scheidebehälter die gewünschte Trennung des eingeleiteten Sumpfproduktes in eine Ober- und eine Unterphase. Auf die unterschiedliche Zusammensetzung dieser beiden Phasen ist bereits weiter oben hingewiesen worden. Der Abzug der schweren Phase (Unterphase) aus dem Scheidebehälter 23 wird dabei durch den Trennschichtregler 24 gesteuert. Dies geschieht in der Weise, dass die Lage der Trennschicht zwischen der schweren und der leichten Phase die Stellung des Trennschichtreglers 24, der an einem Gelenk frei beweglich befestigt ist, beeinflusst. Sobald sich die schwere Phase im unteren Teil des Scheidebehälters 23 so weit angereichert hat, dass sich die Trennschicht zwischen schwerer und leichter Phase auf der gleichen Höhe wie die Trennschichtregler 24 befindet, nimmt dieser die in der Abbildung eingezeichnete waagerechte Stellung ein und betätigt bei Erreichen dieser Stellung über die Impulsleitung 27 den Stellantrieb 28 des Ventils 26 in der Weise, dass dieses geöffnet wird. Da das Ventil 26 in der Leitung 25 installiert ist, wird damit schwere Phase aus dem Scheidebehälter 23 abgezogen und kann über diese Leitung mit dem in der Leitung 3 fliessenden Lösungsmittel vereinigt werden. Sinkt dagegen die Trennschicht zwischen schwerer und leichter Phase im Scheidebehälter weiter nach unten, so verändert sich die Stellung des Trennschichtreglers 24 entsprechend nach unten und das Ventil 26 wird dadurch in der beschriebenen Art und Weise geschlossen bzw. gedrosselt. Die leichte Phase (Oberphase) wird indessen über die Leitung 18 aus dem Scheidebehälter 23 entfernt und gelangt in den Sumpf der Raffinatdestillationskolonne 19 zurück. In Abweichung von der im Fliessschema dargestellten Schaltung ist es natürlich auch möglich, die durch die Leitung 25 abgezogene schwere Phase nicht mit dem Lösungsmittel in der Leitung 3 zu vereinigen, sondern getrennt von diesem in den Oberteil der Extraktivdestillationskolonne 2 einzuleiten.

Die vorteilhafte Wirkung der erfindungsgemässen Arbeitsweise wird durch den nachfolgenden Vergleichsversuch belegt. Als Einsatzprodukt wurde dabei eine aus hydriertem Pyrolysebenzin gewonnene Rohbenzolfraktion verwendet, die einer Extraktivdestillation mit N-Formylmorpholin unterworfen wurde. Der Nichtaromatengehalt im Einsatzprodukt lag bei 25 Gew.-% und der Methylcyclohexangehalt bei 0,2 Gew.-%. Das Methylcyclohexan ist hierbei die den Energieverbrauch in der Extraktivdestillation bestimmende Schlüsselkomponente, da eine Erhöhung des Methylcyclohexangehaltes im Einsatzprodukt um 0,1 Gew.-% erfahrungsgemäss bei der Extraktivdestillation einen Mehrverbrauch an Wärmeenergie von 90 kJ pro kg Einsatzprodukt erforderlich macht.

Der Lösungsmittelgehalt im Kopfprodukt der Extraktivdestillation lag bei 2 Gew.-%. Es ist deshalb erforderlich, dieses Kopfprodukt zum Zwecke der Lösungsmittelrückgewinnung einer Destillation zu unterwerfen. Im vorliegenden Vergleichsversuch wurde im ersten Teil des Vergleichsversuches das Sumpfprodukt dieser sogen. Raffinatdestillation unmittelbar, das heisst ohne Phasentrennung, in die Extraktivdestillationskolonne zurückgeführt, während im zweiten Teil dieses Vergleichsversuches erfindungsgemäss mit Phasentrennung gearbeitet und nur die im Scheidebehälter abgetrennte schwere Phase in die Extraktivdestillationskolonne zurückgeführt wurde. In der nachfolgenden Tabelle sind die wichtigsten Daten der beiden in die Extraktivdestillationskolonne zurückgeführten Produktströme gegenübergestellt.

*(Tabelle auf der nächsten Seite)*

Ein Vergleich der vorliegenden Zahlen zeigt, dass durch die Anwendung des erfindungsgemässen Verfahrens mit Phasentrennung die zur Extraktivdestillationskolonne zurückgeführte Menge an Methylcyclohexan von 1,6 kg auf 0,15 kg verringert wird. Dadurch reduziert sich bei der erfindungsgemässen Arbeitsweise der Wärmeverbrauch in der Extraktivdestillation auf 85% des bei der bisherigen Arbeitsweise erforderlichen Wertes. Ausserdem ist zu berücksichtigen, dass bei der erfindungsgemässen Arbeitsweise der Anteil der übrigen in die Extraktivdestillationskolonne zurückgeführten Nichtaromaten ebenfalls wesentlich niedriger liegt, so dass durch einen zu hohen Nichtaromatenrückfluss bedingte Störungen beim Betrieb der Extraktivdestillationskolonne ausgeschlossen sind.

| | Ohne Phasen-trennung | Mit Phasen-trennung |
|---|---|---|
| Nichtaromaten | 58,7 Gew.-% | 8,5 Gew.-% |
| davon Methylcyclohexan | 12,1 Gew.-% | 2,6 Gew.-% |
| Aromaten | 1,0 Gew.-% | 1,0 Gew.-% |
| N-Formylmorpholin | 40,3 Gew.-% | 90,5 Gew.-% |
| Rückführungsmenge | | |
| pro 1000 kg Einsatz | 13,2 kg | 5,9 kg |
| davon Methylcyclohexan | 1,6 kg | 0,15 kg |

## Patentansprüche

1. Verfahren zur Abtrennung von Aromaten aus Kohlenwasserstoffgemischen beliebigen Aromatengehaltes, die als nichtaromatische Bestandteile insbesondere Paraffine, Cycloparaffine. Olefine, Diolefine sowie organische Schwefelverbindungen enthalten können, durch Extraktivdestillation, bei der N-substituierte Morpholine, deren Substituenten nicht mehr als sieben C-Atome aufweisen, als selektives Lösungsmittel verwendet werden, wobei die nichtaromatischen Bestandteile des als Einsatzprodukt dienenden Kohlenwasserstoffgemisches als Raffinat über Kopf aus der Extraktivdestillationskolonne abdestilliert werden, während die Aromaten zusammen mit dem verwendeten Lösungsmittel als Extrakt aus dem Sumpf der Extraktivdestillationskolonne abgezogen werden und wobei das Raffinat zwecks Rückgewinnung der in ihm vorhandenen Lösungsmittelreste destilliert wird, dadurch gekennzeichnet, dass das bei der Raffinatdestillation anfallende Sumpfprodukt mit einem Lösungsmittelgehalt von 20 bis 75 Gew.-% aus der Raffinatdestillationskolonne abgezogen und unter Abkühlung bis auf eine Temperatur von 20 bis 70° C in einen Scheidebehälter eingeleitet und dort in eine schwere und eine leichte Phase getrennt wird, worauf die schwere Phase in die Extraktivdestillationskolonne und die leichte Phase in die Raffinatdestillationskolonne wiedereingeleitet wird.

2. Verfahren nach Anspruch 1, dadurch gekennzeichnet, dass das bei der Raffinatdestillation anfallende Sumpfprodukt vor dem Eintritt in den Scheidebehälter durch einen Kühler geleitet wird.

3. Verfahren nach den Ansprüchen 1 und 2, dadurch gekennzeichnet, dass die aus dem Scheidebehälter abgezogene schwere Phase gemeinsam mit dem Lösungsmittel in den Oberteil der Extraktivdestillationskolonne wiedereingeleitet wird.

4. Verfahren nach den Ansprüchen 1 und 2, dadurch gekennzeichnet, dass die aus dem Scheidebehälter abgezogene schwere Phase getrennt vom Lösungsmittel in den Oberteil der Extraktivdestillationskolonne wiedereingeleitet wird.

5. Verfahren nach den Ansprüchen 1 bis 4, dadurch gekennzeichnet, dass die aus dem Scheidebehälter abgezogene leichte Phase in den Sumpf der Raffinatdestillationskolonne wiedereingeleitet wird.

6. Verfahren nach den Ansprüchen 1 bis 5, dadurch gekennzeichnet, dass der Abzug der schweren Phase aus dem Scheidebehälter über ein Ventil gesteuert wird, dessen Stellung durch einen im Scheidebehälter angeordneten Trennschichtregler beeinflusst wird.

## Claims

1. Process for separating aromatics from hydrocarbon mixtures of any aromatics content, which can contain especially paraffins, cycloparaffins, olefines, diolefines and organic sulphur compounds as the non-aromatic constituents, by extractive distillation in which N-substituted morpholines, the substituents of which do not have more than seven C atoms, are used as the selective solvent, the non-aromatic constituents of the hydrocarbon mixture used as the feed material being distilled off as a raffinate over the top of the extractive distillation column, whilst the aromatics are taken off, together with the solvent used, as the extract from the bottom of the extractive distillation column, and the raffinate being distilled for recovery of the solvent residues present therein, characterised in that the bottom product obtained in the raffinate distillation is taken off from the raffinate distillation column at a solvent content of 20 to 75% by weight and, with cooling down to a temperature of 20 to 70° C, is introduced into a separator vessel and separated therein into a heavy phase and a light phase, whereupon the heavy phase is re-introduced into the extractive distillation column and the light phase is re-introduced into the raffinate distillation column.

2. Process according to Claim 1, characterised in that the bottom product obtained in the raffinate distillation is passed through a cooler before it enters the separator vessel.

3. Process according to Claims 1 and 2, characterised in that the heavy phase taken off from the separator vessel is re-introduced together with the solvent into the upper section of the extractive distillation column.

4. Process according to Claims 1 and 2, characterised in that the heavy phase taken off from the separator vessel is introduced separately from the solvent into the upper section of the extractive distillation column.

5. Process according to Claims 1 to 4, characterised in that the light phase taken off from the separator vessel is re-introduced into the bottom of the raffinate distillation column.

6. Process according to Claims 1 to 5, characterised in that the take-off of the heavy phase from the separator vessel is controlled by a valve, the position of which is adjusted by means of an interface controller provided in the separator vessel.

## Revendications

1. Procédé pour séparer les fractions aromatiques de mélanges hydrocarbonés d'une teneur quelconque en fractions aromatiques et pouvant renfermer, comme constituants non aromatiques, en particulier des paraffines, des cycloparaffines, des oléfines, des dioléfines, ainsi que des composés organiques soufrés, par une distillation extractive, lors de laquelle on utilise, comme solvant sélectif, des morpholines N-substituées, dont les substituants ne présentent pas plus de sept atomes de carbone, tandis que les constituants non aromatiques du mélange hydrocarboné servant de produit de départ sont séparés par distillation en tant que raffinat à la tête de la colonne de distillation extractive, alors que les fractions aromatiques, conjointement avec le solvant utilisé, sont soutirées en tant qu'extrait à la base de la colonne de distillation extractive et que le raffinat, en vue de récupérer les restes de solvant qu'il renferme, est distillé, caractérisé par le fait que le produit de queue se formant lors de la distillation du raffinat et présentant une teneur en solvant de 20 à 75% en poids, est soutiré de la colonne de distillation du raffinat et qu'en étant refroidi jusqu'à une température de 20 à 70° C, il est introduit dans un récipient séparateur où il est séparé en une phase lourde et en une phase légère, la phase lourde étant ensuite réintroduite dans la colonne de distillation extractive et la phase légère dans la colonne de distillation du raffinat.

2. Procédé selon la revendication 1, caractérisé par le fait que le produit de queue se formant lors de la distillation du raffinat est amené à passer par un réfrigérant avant de pénétrer dans le récipient séparateur.

3. Procédé selon les revendications 1 et 2, caractérisé par le fait que la phase lourde soutirée du récipient séparateur est renvoyée, en commun avec le solvant, dans la partie supérieure de la colonne de distillation extractive.

4. Procédé selon les revendications 1 et 2, caractérisé par le fait que la phase lourde, soutirée du récipient de séparation, est renvoyée séparément du solvant dans la partie supérieure de la colonne de distillation extractive.

5. Procédé selon les revendications 1 à 4, caractérisé par le fait que la phase légère, soutirée du récipient séparateur, est renvoyée à la base de la colonne de distillation du raffinat.

6. Procédé selon les revendications 1 à 5, caractérisé par le fait que le soutirage de la phase lourde du récipient séparateur est commandée par une soupape dont la position est influencée par un régulateur de niveau d'interface disposé dans le récipient séparateur.